(19) Europäisches Patentamt
European Patent Office
Office européen des brevets

(11) EP 0 650 962 B1

(12) **FASCICULE DE BREVET EUROPEEN**

(45) Date de publication et mention de la délivrance du brevet:
**03.03.1999 Bulletin 1999/09**

(51) Int. Cl.$^6$: **C07D 233/32**

(21) Numéro de dépôt: **94402412.4**

(22) Date de dépôt: **26.10.1994**

(54) **Procédé de préparation de (méth)acrylates d'alkylimidazolidone**

Verfahren zur Herstellung von Alkylimidazolidon (Meth)acrylat

Process for the preparation of alkylimidazolidone (meth)acrylate

(84) Etats contractants désignés:
**AT BE CH DE DK ES FR GB GR IE IT LI LU NL PT SE**

(30) Priorité: **27.10.1993 FR 9312833**

(43) Date de publication de la demande:
**03.05.1995 Bulletin 1995/18**

(73) Titulaire: **ELF ATOCHEM S.A.**
**92800 Puteaux, Hauts-de-Seine (FR)**

(72) Inventeurs:
- **Riondel, Alain**
  **F-57600 Forbach (FR)**
- **Herbst, Gilles**
  **F-57350 Spicheren (FR)**
- **Esch, Marc**
  **F-57800 Freyming-Merlebach (FR)**

(74) Mandataire: **Chaillot, Geneviève**
**Cabinet CHAILLOT,**
**16-20, avenue de L'Agent Sarre,**
**B.P. 74**
**92703 Colombes Cédex (FR)**

(56) Documents cités:
**EP-A- 0 433 135** **EP-A- 0 571 851**

EP 0 650 962 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Xerox (UK) Business Services
2.16.7/3.6

## Description

La présente invention porte sur un procédé de fabrication d'un composé de formule:

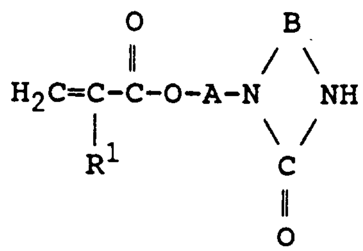

(I)

dans laquelle :

- $R^1$ représente hydrogène ou méthyle ; et
- A et B représentent chacun indépendamment une chaîne hydrocarbonée droite ou ramifiée, ayant de 2 à 5 atomes de carbone,

par réaction d'au moins un (méth)acrylate de formule :

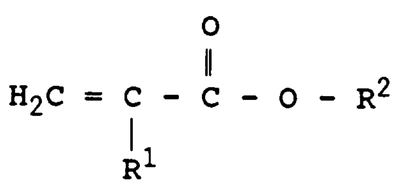

(II)

dans laquelle :

- $R^1$ a la signification précitée ; et
- $R^2$ représente un groupe alkyle en $C_1$-$C_4$,

avec un alcool hétérocyclique de formule :

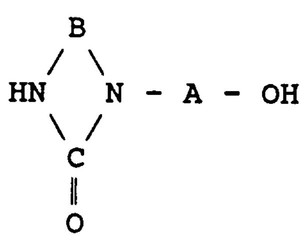

(III)

dans laquelle A et B ont les significations précitées.

Ces composés de formule (I) sont connus pour leur rôle dans la constitution de polymères utiles comme revêtements et adhésifs, pour le traitement du papier et des textiles, notamment par le brevet américain US-A-2 871 223, ainsi que pour leurs utilisations comme agents de traitement du cuir, et dans la production de peintures en émulsion. Le méthacrylate d'éthylimidazolidone (MEIO) est principalement utilisé dans les peintures et en tant que promoteur d'adhésion humide.

Dans le procédé défini ci-dessus, connu par la demande de brevet européen EP-A-0 236 994, les catalyseurs sont choisis parmi les alcoolates de titanes, comme les titanates de tétralkyle, et les chélates de Ti, Zr, Fe ou Zn avec des composés 1,3-dicarbonyle comme les acétylacétonates de Ti, Zr, Fe ou Zn.

On peut également souligner que, dans cette demande européenne EP-A-0 236 994, est signalée l'utilisation connue de catalyseurs basiques KOH, $K_2CO_3$, MeONa, dérivés de pyrimidine, avec la recommandation de ne pas les

utiliser en raison de leur forte propension à favoriser les réactions secondaires.

Il est également connu, par la demande de brevet européen EP-A-0 433 135, d'utiliser, comme catalyseurs, pour cette même réaction, les dialkyloxydes d'étain, les dialkyldialcoxydes d'étain et les dialkyldiesters d'étain. On cite, entre autres, le di-n-butyloxyde d'étain (DBTO).

Cependant, dans le cas de la synthèse du MEIO, on cherche à atteindre une conversion la plus importante possible de l'hydroxyéthylimidazolidone (HEIO), ce qui, dans le cas d'une catalyse par le DBTO, nécessite un niveau thermique élevé.

On a donc recherché un catalyseur autre permettant notamment d'obtenir une bonne productivité et une bonne sélectivité à des températures de réaction moins élevées. Il a maintenant été découvert de façon surprenante que l'utilisation d'un alcoolate de magnésium permet d'opérer à une température inférieure à 100°C (95°C-96°C notamment), tout en conduisant à de bons résultats du point de vue du rendement en MEIO et de la conversion de l'HEIO.

La présente invention a donc pour objet le procédé de fabrication d'un composé de formule (I), tel qu'il a été défini ci-dessus, en présence d'au moins un alcoolate de magnésium en tant que catalyseur.

A titre d'exemples d'alcoolates de magnésium $Mg(OR)_2$, on peut citer ceux pour lesquels R représente un groupe alkyle linéaire en $C_1$-$C_4$, tel que méthyle, éthyle, n-propyle, n-butyle. On peut citer plus particulièrement les alcoolates pour lesquels R représente éthyle ou n-propyle.

On préfère utiliser le diéthylate de magnésium.

Comme exemples de réactifs de formule (II), on peut citer notamment les acrylates et méthacrylates de méthyle, d'éthyle, de n-propyle, de n-butyle et d'isobutyle.

Comme exemple d'alcool hétérocyclique de formule (III), on peut citer, notamment, la 1-(2-hydroxyéthyl)-imidazolidyl-2-one (HEIO).

La quantité de catalyseur utilisée pour la mise en oeuvre du procédé selon l'invention est généralement comprise entre 0,5 et 4% environ en moles, et de préférence, entre 1 et 2,5% environ en moles, par rapport à l'alcool hétérocyclique de formule (III).

La réaction du procédé selon l'invention peut être effectuée en présence d'un excès de l'un ou l'autre des réactifs. Il est toutefois conseillé que le rapport molaire (méth)acrylate de formule (II)/alcool hétérocyclique de formule (III) soit compris entre 1,1 et 7,0 environ, de préférence entre 2,0 et 6,0. En opérant avec un large excès molaire de (méth)acrylate par rapport à l'alcool hétérocyclique, on obtient, à l'issue de la réaction, une solution de composé de formule (I) dans le (méth)acrylate qui peut être utilisée directement pour certaines applications, telles que l'obtention de peintures et revêtements ou bien le traitement du cuir.

La réaction du procédé selon l'invention est, de préférence, effectuée en présence d'au moins un inhibiteur de polymérisation, utilisé, par exemple, à raison de 0,05 à 0,5% en poids sur la base du poids de l'alcool hétérocyclique de formule (III). Comme exemples d'inhibiteurs de polymérisation utilisables, on peut citer notamment la phénothiazine, l'éther méthylique de l'hydroquinone, le di-tertiobutylcatéchol, l'hydroquinone, le p-anilinophénol, la paraphénylène diamine et leurs mélanges en toutes proportions.

La réaction du procédé selon l'invention est effectuée, de préférence, sous une pression ne dépassant pas la pression atmosphérique, par exemple une pression comprise entre 0,3 et 1 bar. De façon avantageuse, la réaction est réalisée sous bullage d'air pour améliorer l'efficacité des stabilisants. Elle est effectuée en mélangeant le (méth)acrylate de formule (II) et l'alcool hétérocyclique de formule (III), et en chauffant le mélange réactionnel au reflux, généralement à une température comprise entre 85 et 105°C, cette température étant évidemment dépendante de la nature exacte de l'alcool et du (méth)acrylate, et du système catalytique mis en oeuvre.

Dans la mise en oeuvre du procédé selon l'invention, il est conseillé d'atteindre une déshydratation maximale avant l'addition du catalyseur, de manière à éviter la désactivation de ce dernier par l'eau. On peut parvenir à ce résultat, par exemple, en chauffant le mélange initial de (méth)acrylate de formule (II), d'alcool hétérocyclique de formule (III) et d'inhibiteur de polymérisation au reflux, tout en séparant par distillation l'azéotrope de (méth) acrylate et d'eau lorsqu'il se forme un azéotrope de méthacrylate et d'eau. A ce stade, après séparation du distillat, le catalyseur est introduit dans le mélange réactionnel à chaud.

La durée de la réaction selon l'invention, dépend évidemment de conditions réactionnelles, telles que la température, la pression et la quantité de catalyseur utilisée, mais est généralement comprise entre 3 et 15 heures environ. Elle dépend évidemment aussi de la nature des réactifs mis en oeuvre.

Le mélange réactionnel est donc chauffé au reflux jusqu'à ce que la température de tête atteigne la température de distillation de l'azéotrope du (méth)acrylate et de l'alcool de formule $R_2OH$ formé par la réaction lorsqu'il se forme un azéotrope.

L'excès éventuel de (méth)acrylate peut ensuite être éliminé par évaporation, de manière à isoler le composé de formule (I) du milieu réactionnel, généralement à l'état solide : ainsi, l'acrylate de 1-(2-hydroxyéthyl) imidazolidyl-2-one est un solide blanc cristallin de température de fusion égale à 43°C, soluble à froid dans les cétones, les alcools, les hydrocarbures aromatiques et l'eau, insoluble à froid dans les hydrocarbures saturés et qui précipite à 0°C dans l'acrylate d'éthyle. Le méthacrylate de 1-(2-hydroxyéthyl) imidazolidyl-2-one est un solide blanc cristallin de température de fusion égale à 47°C, possédant les mêmes propriétés de solubilité que l'acrylate précédent. A l'issue de l'opération d'évaporation, le produit solide cristallin peut en outre être purifié par filtration puis lavage à l'aide d'éther de pétrole, et séchage.

L'isolement du composé (I) peut aussi être réalisé par évaporation partielle du (méth)acrylate, puis cristallisation à une température suffisamment basse (de préférence inférieure ou égale à 0°C) et pendant une durée suffisamment longue (pouvant atteindre jusqu'à 15 heures), puis filtration suivie des étapes de purification décrites ci-dessus.

Enfin, une troisième méthode pour isoler le composé de formule (I) de la solution le contenant, consiste à effectuer une extraction par l'eau, suivie d'une décantation, d'une concentration du (méth)acrylate et des étapes de purifi-cation décrites ci-dessus.

Les exemples suivants illustrent l'invention sans toutefois la limiter. Dans ces exemples, les pourcentages sont indiqués en poids sauf indication contraire.

EXEMPLES

Exemple 1 :

Dans un réacteur en verre, à double enveloppe, équipé d'une sonde de mesure de la température, d'un agitateur mécanique à vitesse variable, d'une colonne adiabatique à garnissage, surmontée d'une tête de reflux, on introduit 195 g d'HEIO et 565 g de méthacrylate de méthyle (MAM), ainsi que 0,36 g de phénothiazine (PTZ) en tant que stabilisant. On met en service une stabilisation en tête de colonne par une solution à 0,2% d'éther méthylique de l'hydroquinone (EMHQ) dans le MAM. On porte le contenu du réacteur à ébullition sous pression atmosphérique pendant 1 heure, à une température de tête de colonne de 98-100°C et une température de pied de colonne inférieure ou égale à 100°C, et on élimine l'eau par distillation azéotropique avec le méthacrylate de méthyle.

Ensuite, on introduit dans le réacteur 3,4 g d'éthylate de magnésium et du MAM en veillant que le rapport molaire MAM/HEIO soit égal à 3,5 (après séchage). On ajuste la pression pour maintenir dans le réacteur une température T de 96°C. On régule le soutirage de l'azéotrope MAM/MeOH par une température de consigne en tête de colonne (égale à 65°C). Lorsque la quantité de méthanol soutirée correspond à la quantité attendue, on prolonge la réaction jusqu'à ce que l'on ne constate plus de formation de méthanol (température en tête de colonne = température d'ébullition du MAM ), à reflux total, sous la pression considérée.

Après refroidissement, on récupère du MEIO brut.

Le rendement en MEIO et la conversion de l'HEIO sont déterminés d'après l'analyse par chromatographie en phase liquide (HPLC) du brut réactionnel, par les équations suivantes :

$$\text{Conversion C HEIO (\%)} = \frac{\text{(HEIO de départ - HEIO final)}}{\text{HEIO de départ}} \times 100$$

$$\text{Rendement R MEIO (\%)} = \frac{\text{Nombre de moles de MEIO formé}}{\text{Nombre de moles d'HEIO de départ}} \times 100$$

Les résultats sont rapportés dans le Tableau ci-après, lequel comprend également les données et résultats de quatre autres Exemples.

Tableau

| Ex. | Rapport molaire [MAM]/[HEIO] | Analyse HPLC du mélange brut obtenu (%) | | | T (°C) | R (%) | C (%) |
|---|---|---|---|---|---|---|---|
| | | MAM | HEIO | MEIO | | | |
| 1 | 3,5 | 44,1 | 0,7 | 42,2 | 96 | 80 | 97 |
| 2 | 5,2 | 64,2 | 0,68 | 34,6 | 96 | 87 | 97,2 |
| 3* | 5,2 | 57,5 | 0,42 | 33,3 | 96 | 85 | 97,7 |
| 4 | 3,5 | 45,2 | 4,6 | 40,3 | 90 | 74,5 | 87 |
| 5 | 2,7 | 40,5 | 1,4 | 46,9 | 96 | 77,8 | 96,5 |

* Le catalyseur utilisé dans cet exemple est le propylate de magnésium (le catalyseur utilisé dans les Exemples 2, 4 et 5 est le même quà l'Exemple 1).

## Revendications

1. Procédé de fabrication d'un composé de formule:

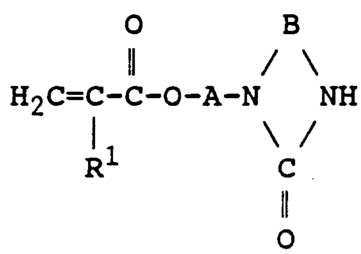

(I)

dans laquelle :

- $R^1$ représente hydrogène ou méthyle ; et
- A et B représentent chacun indépendamment une chaîne hydrocarbonée droite ou ramifiée, ayant de 2 à 5 atomes de carbone,

par réaction d'au moins un (méth)acrylate de formule :

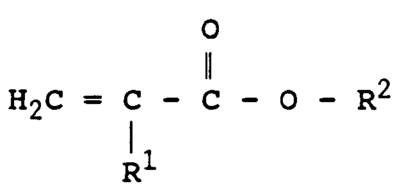

(II)

dans laquelle :

- $R^1$ a la signification précitée ; et
- $R^2$ représente un groupe alkyle en $C_1$-$C_4$,

avec un alcool hétérocyclique de formule :

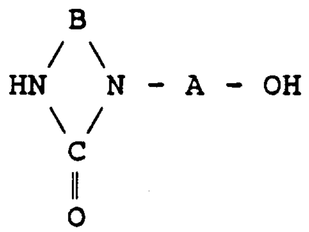

(III)

dans laquelle A et B ont les significations précitées,
en présence d'au moins un alcoolate de magnésium en tant que catalyseur.

**2.** Procédé selon la revendication 1, caractérisé par le fait que l'on utilise un alcoolate de magnésium $Mg(OR)_2$, R représentant un reste alkyle en $C_1$-$C_4$.

**3.** Procédé selon la revendication 2, caractérisé par le fait que R représente éthyle ou n-propyle.

**4.** Procédé selon l'une quelconque des revendications 1 à 4, caractérisé par le fait que l'on utilise le catalyseur dans une quantité de 0,5 à 4% en moles par rapport à l'alcool hétérocyclique de formule (III).

**5.** Procédé selon l'une des revendications 1 à 4, caractérisé par le fait que l'on conduit la réaction à une température comprise entre 85 et 105°C.

**6.** Procédé selon l'une des revendications 1 à 5, caractérisé par le fait que l'on utilise un rapport molaire du (méth)acrylate de formule (II) à l'alcool hétérocyclique de formule (III) qui est compris entre 1,1 et 7,0.

**7.** Procédé selon l'une des revendications 1 à 6, caractérisé par le fait que l'on conduit la réaction pendant une durée comprise entre 3 à 15 heures, sous une pression ne dépassant pas la pression atmosphérique.

**8.** Procédé selon l'une des revendications 1 à 7, caractérisé par le fait que l'on conduit la réaction en présence d'au moins un inhibiteur de polymérisation choisi parmi la phénothiazine, l'éther méthylique de l'hydroquinone, le di-tertiobutylcatéchol, l'hydroquinone, le p-anilinophénol, la paraphénylène diamine et leurs mélanges en toutes proportions.

**Claims**

**1.** Process for the production of a compound of formula:

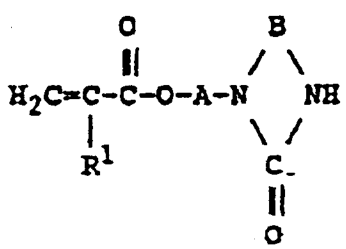

(I)

in which:

- $R^1$ represents hydrogen or methyl; and
- A and B each independently represent a straight or branched hydrocarbon chain having from 2 to 5 carbon atoms,

by reaction of at least one (meth)acrylate of formula:

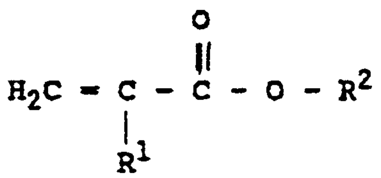

(II)

in which:

- $R^1$ has the above meaning; and
- $R^2$ represents a $C_1$-$C_4$ alkyl group,

with a heterocyclic alcohol of formula:

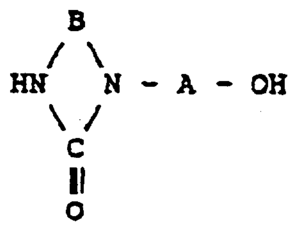

(III)

in which A and B have the above meanings,
in the presence of at least one magnesium alkoxide as catalyst.

2. Process according to Claim 1, characterized in that a magnesium alkoxide $Mg(OR)_2$ is used, R representing a $C_1$-$C_4$ alkyl residue.

3. Process according to Claim 2, characterized in that R represents ethyl or n-propyl.

4. Process according to any one of Claims 1 to 4, characterized in that the catalyst is used in an amount of 0.5 to 4 mol% relative to the heterocyclic alcohol of formula (III).

5. Process according to one of Claims 1 to 4, characterized in that the reaction is carried out at a temperature between 85 and 105°C.

6. Process according to one of Claims 1 to 5, characterized in that a molar ratio of the (meth)acrylate of formula (II) to the heterocyclic alcohol of formula (III) which is between 1.1 and 7.0 is used.

7. Process according to one of Claims 1 to 6, characterized in that the reaction is carried out for a period of between 3 and 15 hours and at a pressure not exceeding atmospheric pressure.

8. Process according to one of Claims 1 to 7, characterized in that the reaction is carried out in the presence of at least one polymerization inhibitor chosen from phenothiazine, hydroquinone methyl ether, di-tert-butylcatechol, hydroquinone, p-anilinophenol, para-phenylenediamine and their mixtures in all proportions.

## Patentansprüche

1. Verfahren zur Herstellung einer Verbindung der Formel:

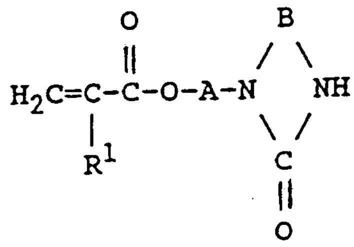 (I),

worin bedeuten:

- $R^1$ Wasserstoff oder Methyl; und
- A und B unabhängig voneinander eine geradkettige oder verzweigte Kohlenwasserstoffkette mit 2 bis 5 Kohlenstoffatomen,

durch Umsetzung mindestens eines (Meth)acrylats der Formel

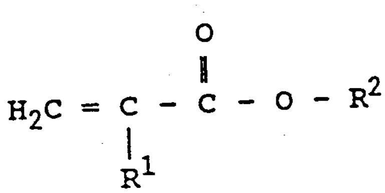 (II),

worin bedeuten:

- $R^1$ die oben angegebene Bedeutung; und
- $R^2$ eine $C_{1-4}$-Alkylgruppe,

mit einem heterocyclischen Alkohol der Formel:

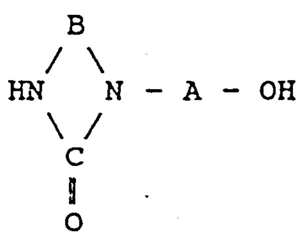 (III),

worin A und B die oben angegebenen Bedeutungen aufweisen,
in Gegenwart mindestens eines Magnesiumalkoholats als Katalysator.

**2.** Verfahren nach Anspruch 1, dadurch gekennzeichnet, daß ein Magnesiumalkoholat $Mg(OR)_2$ verwendet wird, worin R eine $C_{1-4}$-Alkylgruppe bedeutet.

**3.** Verfahren nach Anspruch 2, dadurch gekennzeichnet, daß R Ethyl oder n-Propyl bedeutet.

**4.** Verfahren nach einem der Ansprüche 1 bis 3, dadurch gekennzeichnet, daß der Katalysator in einem Mengenanteil von 0,5 bis 4 Mol-%, bezogen auf den heterocyclischen Alkohol der Formel (III), verwendet wird.

**5.** Verfahren nach einem der Ansprüche 1 bis 4, dadurch gekennzeichnet, daß die Umsetzung bei einer Temperatur im Bereich von 85 bis 105 °C durchgeführt wird.

6. Verfahren nach einem der Ansprüche 1 bis 5, dadurch gekennzeichnet, daß ein Molverhältnis von (Meth)acrylat der Formel (II) zu heterocyclischem Alkohol der Formel (III) im Bereich von 1,1 bis 7,0 verwendet wird.

7. Verfahren nach einem der Ansprüche 1 bis 6, dadurch gekennzeichnet, daß die Umsetzung während einer Zeitdauer von 3 bis 15 h bei einem Druck nicht über Atmosphärendruck durchgeführt wird.

8. Verfahren nach einem der Ansprüche 1 bis 7, dadurch gekennzeichnet, daß die Umsetzung in Gegenwart mindestens eines Polymerisationsinhibitors durchgeführt wird, der unter Phenothiazin, Hydrochinonmethylether, Di-tert.-butyl-catechin, Hydrochinon, p-Anilinophenol, p-Phenylendiamin und deren Gemischen in beliebigen Verhältnissen ausgewählt ist.